# EUROPÄISCHE PATENTSCHRIFT

(11) **EP 1 905 377 B1**
(45) Veröffentlichungstag und Bekanntmachung des Hinweises auf die Patenterteilung: **29.05.2013**
(21) Anmeldenummer: 06020435.1
(22) Anmeldetag: 28.09.2006
(51) Int. Cl.: A61B 19/00

(54) **Planung von Bewegungspfaden medizinischer Instrumente**
Preoperative planing of the position of surgical instruments
Planification pré-opératoire des chemins de déplacement de dispositifs chirurgicaux

(43) Veröffentlichungstag der Anmeldung: 02.04.2008
(73) Patentinhaber: BrainLAB AG, 85622 Feldkirchen (DE)
(72) Erfinder: Rodriguez Ponce, Maria Inmaculada, 81669 München (DE)
(74) Vertreter: Engelhard, Maximilian

(56) Entgegenhaltungen:
- WO-A2-02/19243
- DE-A1- 10 252 837
- US-A1- 2004 010 221
- US-A1- 2006 084 860

## Beschreibung

Die vorliegende Erfindung betrifft die Einführung von Instrumenten, insbesondere von Kathetern, Spritzen, Biopsienadeln etc. in eine Körperstruktur, wie z.B. biologisches Gewebe, wie z.B. Organe und/oder Gehirn und/oder Knochen, die eine räumlich sich unterscheidende Steifheitheit ("rigidity") und/oder Penetrierbarkeit und/oder Elastizität aufweisen. Derartige heterogene Körperstrukturen können das Einführen eines Instruments zu der gewünschten Position erschweren oder unmöglich machen. Insbesondere kann das Instrument aufgrund der Heterogenität und der damit bedingten insbesondere mechanischen Wechselwirkung zwischen dem heterogenen Gewebe und dem Instrument eine Ablenkung vom gewünschten und geplanten Bewegungspfad, insbesondere Einführpfad des Instruments bewirken.

Heterogene Körperstrukturen treten insbesondere bei von Tumoren befallenen Körperstrukturen auf, insbesondere bei von Tumoren befallenen biologischen Gewebe, also Organen wie zum Beispiel Gehirn oder Leber. Wie man beispielsweise mithilfe der Elastographie ("elastography") feststellen kann, sind Tumorzellen 5- bis 28-mal steifer (weniger elastisch) als Zellen des gesunden Gewebes. Dies kann mit Ultraschall-Elastographie oder MR-Elastographie gemessen werden. Aufgrund dieser steifen und/oder rigiden Tumorbereiche ergeben sich für den Operateur Risiken, dass er den gewünschten Bereich mit nicht vollständig rigiden Instrumenten wie zum Beispiel einem Katheter nicht erreichen kann. Ursachen können sein, dass das Instrument durch Tumorgewebe von seinem gewünschten Pfad abgelenkt wird, dass Tumorgewebe mit dem verwendeten Instrument nicht durchdrungen werden kann oder dass das Instrument gebogen oder abgelenkt wird, so dass die Ausspritzrichtung zum Beispiel eines Katheters nicht mehr in der geplanten Richtung liegt und somit beispielsweise ein eingespritzte Wirkstoff ohne Effekt verbleibt oder sogar an einer nicht vorhergesehenen Stelle im gesunden Gewebe Schaden verursachen kann. Entsprechendes gilt beispielsweise bei der Verwendung einer Biopsienadel, die aufgrund der Ablenkung der Nadel durch steifere Bereiche des heterogenen Gewebes Proben aus einem falschen Bereich entnimmt, was dann zu Fehlinterpretationen bei der Diagnose durch den Arzt führen kann.

Aus US 2004/0010221 A1 ist ein Verfahren und eine Vorrichtung zum Vorbereiten einer Drainage bekannt. Ein Katheter wird so positioniert, dass wenigstens ein Teil der Körperflüssigkeit abgeführt werden kann. Hierzu wird die anatomische Struktur des Körpers berücksichtigt.

Aus US 2006/0084860 A1 ist ein Verfahren für eine virtuelle Endoskopie bekannt. Dabei wird die Biegungscharakteristik des Endoskops berücksichtigt.

Aufgabe dieser Erfindung ist es, ein Verfahren und eine Vorrichtung, insbesondere ein Planungsverfahren und ein Planungsprogramm einem Bediener (z.B. Arzt, insbesondere Operateur) zur Verfügung zu stellen, um einen Eingriff in eine heterogeneKörperstruktur mithilfe eines Instruments besser planen zu können, so dass die Wahrscheinlichkeit erhöht wird, dass ein geplanter Eingriff dieser Planung gemäß ausgeführt werden kann.

Vorstehende Aufgabe wird durch die Gegenstände der unabhängigen Ansprüche gelöst. Die abhängigen Ansprüche sind auf vorteilhafte Weiterbildungen gerichtet.

Vorteilhaft wird ein Verfahren bereitgestellt, das es erlaubt, Bereiche in einer Körperstruktur, insbesondere in einer heterogenen Körperstruktur zu analysieren in Hinblick auf das Risiko der Ablenkung oder des Biegens eines Instrumentes, zum Beispiel eines Katheters (insbesondere eines Infusionskatheters) beim Einführen dieses Instrumentes oder Katheters in oder durch das betreffende Gewebe. Dabei greift man insbesondere auf Informationen zurück, die Aussagen über die elastischen Eigenschaften der heterogenen Körperstruktur, insbesondere des Zielgebiets, also des Gewebes am Zielort erlauben. Der Zielort ist derjenige Ort, der durch das Instrument erreicht werden soll. Weiter kann vorteilhaft Information wie z.B. die Verteilung des Druckes, der Spannungen, der Dehnungen, der Verschiebungen und Verzerrrungen der heterogenen Körperstruktur, insbesondere des heterogenen biologischen Gewebes verwendet werden. Ein Beispiel stellt der lokale oder nicht lokale interstitielle Druck dar. Weiter werden vorzugsweise Informationen über das Instrument, insbesondere die mechanischen Eigenschaften des Instruments verwendet. Dies kann aus einer Datenbank abgelesen werden, in der für den Instrumententyp (z.B. Kathetertyp oder Nadeltyp) mechanische Informationen verzeichnet sind. Dazu zählen insbesondere die geometrischen Abmessungen , die Elastizität bzw. Steifigkeit, das Material des Instruments usw.

Alle oder ein Teil der vorgenannten Informationen können verwendet werden, um das Risiko des Biegens oder Ablenkens des Instruments in der anisotropen Körperstruktur zu berechnen oder das Risiko zu berechnen, den Zielbereich durch das Instrument, insbesondere durch ein gewähltes Instrument nicht erreichen zu können. Beispielsweise kann der Weg zu dem Zielbereich durch einen für das Instrument undurchdringlichen Bereich der heterogenen Körperstruktur versperrt sein. Vorzugsweise werden Bereiche hohen und niedrigen Risikos des Biegens, Ablenkens und/oder der Blockade des Instruments beim Einführen angezeigt. Vorzugsweise werden Informationen über die mechanischen Eigenschaften der heterogenen Körperstruktur, insbesondere deren elastischen Eigenschaften und Steifigkeitseigenschaften räumlich aufgelöst oder gemittelt für größere Bereiche oder insgesamt angegeben und vom erfindungsgemäßen Verfahren verwendet.

Vorteilhaft werden die mechanischen Eigenschaften der heterogenen Körperstruktur, insbesondere im Zielbereich ausgedrückt durch z.B. elastische Konstanten, Schermodul, Elastizitätsmodul bzw. Youngmodul, Dehnungskonstanten, Härtegrade der Oberflächen rigider Strukturen usw. Die vorgenannten Größen stellen Beispiele dar zur Beschreibung der mechanischen Eigenschaften und können einzeln oder in Kombination verwendet werden.

Informationen über die elastischen Eigenschaften können beispielsweise über Magnetresonanzverfahren (MR-Verfahren), Ultraschalltechniken oder invasive, In-vitro- oder nicht invasive Verfahren durchgeführt werden, so wie aus der Literatur entnommen werden. Beispielsweise können Testsonden in das zu untersuchende Gewebe oder in von Tumoren befallene Proben eingeführt werden und deren Ablenkung kann insbesondere bilddiagnostisch erfasst werden. Dies gilt allgemein auch für die Bestimmung anderer mechanischer Eigenschaften des heterogenen Gewebes. Ebenfalls können In-vitro-Verfahren verwendet werden, um die mechanischen Eigenschaften des betrachteten Gewebes, wie elastische Eigenschaften und Penetrationsfähigkeit zu bestimmen.

Mechanische Eigenschaften des Instruments können ebenfalls durch die Beschreibung der materiellen Zusammensetzung des Instruments und der verwendeten Materialien bestimmt werden. In Datenbanken können die Eigenschaften anhand des kommerziellen Namens des Instruments, z.B. Kathetertyps nachgeschlagen werden. Ebenso können die mechanischen Eigenschaften des Instruments durch entsprechende Tests ermittelt werden.

Bevorzugt wird das Risiko des Biegens, Ablenkens oder der Nichtdurchdringung eines Bereiches mittels des Instruments basierend auf mathematischen Gleichungen berechnet, die die mechanischen Eigenschaften der heterogenen Körperstruktur, insbesondere im Zielbereich beschreiben. Dazu können die mathematischen Gleichungen insbesondere den Hintergrunddruck ("background pressure") in dem betrachteten Bereich der heterogenenKörperstruktur, insbesondere den Zielbereich verwenden und diesen insbesondere zu einem Druck in Beziehung zu setzen, der von dem Instrument in dem betrachteten Bereich der heterogenen Körperstruktur, insbesondere dem Zielbereich ausgeübt wird.

Der Hintergrunddruck umfasst insbesondere den natürlichen Druck oder stellt den natürlichen Druck dar, der durch das Gewebe und seinen biologischen Fluidinhalt gebildet wird. Beim gesunden Gehirn liegt der Druck typischerweise bei 1 bis 4 mmHg. Das Druckprofil kann stark heterogen sein, insbesondere im nichtgesunden Gewebe, wie z.B. Tumor. Beispielsweise ist der Druck im Kern eines Tumors stark unterschiedlich von dem Druck an den Rändern des Tumors. Falls ein Katheter diesen Bereich quert oder durchdringt, wird er somit einen starken Druckradienten erfahren, der ihn zum Biegen bringen kann. Ein räumliches Druckprofil über das Gewebe erlaubt die Bestimmung des Druckgradienten und somit die Bestimmung von Kräften, die ein Biegen des Katheters bewirken können. In vitro Experimente können verwendet werden, um ein Druckprofil innerhalb eines Tumors zu bestimmen.

Alternativ oder ergänzend können entsprechende Kräfte, Drehmomente und/oder Energien, die in dem betrachteten Bereich der heterogenen Körperstruktur vorliegen bzw. dort ausgeübt werden und die von dem Instrument ausgeübt werden für das erfindungsgemäße Verfahren mit herangezogen werden.

Das Risiko des Biegens, Ablenkens oder des Nichtdurchdringens wird vorzugsweise räumlich aufgelöst berechnet, so dass sich beispielsweise eine Verteilung des Ablenkungsrisikos für die betrachtete heterogene Körperstruktur ergibt. Dies kann z.B. farbig auf einer Anzeige, beispielsweise mittels Fehlfarben dargestellt werden.

Insbesondere kann aufgrund der Informationen über die mechanischen Eigenschaften der heterogenen Körperstruktur, die insbesondere räumlich aufgelöst ist, das Risiko der Ablenkung entlang eines vorgesehenen Einführpfades des Instruments (z.B. Kathetertrajektorie) berechnet werden. Dabei oder bei anderen Varianten des erfindungsgemäßen Verfahrens kann berücksichtigt werden, dass die mechanischen Eigenschaften des Instruments heterogen sein können. Also insbesondere nicht homogen und sie sich z.B. abschnittsweise unterscheiden. Beispielsweise kann die Spitze des Instruments eine andere Elastizität aufweisen als der hintere Teil.

Vorzugsweise wird durch das erfindungsgemäße Verfahren eine Risikoverteilung berechnet, die beispielsweise das Ablkenkungs- oder Biegerisiko und/oder Nichtdurchdringungsrisiko für einen vorgesehenen Pfad ("planned trajectory") beschreibt, den das Instrument in der heterogenen Körperstruktur, insbesondere im Zielbereich zurücklegen soll. Auf diese Art und Weise ist es einen Operateur möglich, zu beurteilen, ob der vorgesehene Pfad zu riskant oder erfolgsversprechend ist. Die Angaben über das Risiko der Ablenkung oder des Biegens und dessen räumliche Verteilung in der heterogenen Körperstruktur, insbesondere entlang des geplanten Pfades kann sich auch auf einen Teil des Instruments beschränken, insbesondere dessen Spitze. Dies ist insbesondere dann von Interesse, falls mittels Instrument ein Ausspritzen, z.B. eines Wirkstoffes im Zielbereich erfolgen soll. In diesem Fall ist dann die Ausrichtung der Ausspritzöffnung für den Operateur entscheidend, um beurteilen zu können, ob er den gewünschten Bereich mit dem Wirkstoff erreicht oder nicht.

Allgemein betrifft die Erfindung ein Verfahren zum Bestimmen einer Lage eines Instruments, insbesondere eines flexiblen Instruments, insbesondere elastischen Instruments. Das Instrument hat die Eigenschaft, dass es durch mechanische Eigenwirkungen seine Form ändern kann, sich insbesondere biegen kann. Andere Beispiele für mechanische Formveränderungen sind das Stauchen und/oder Dehnen des Instruments und/oder Knicken des Instruments.

Die vorgenannte Lage des Instruments umfasst insbesondere die Lage des Instruments im Raum, also insbesondere Lage des Bereichs, den das das Instrument im Raum einnimmt, die Orientierung des Instruments, die Ausrichtung des Instruments, insbesondere die Ausrichtung des aktiven Instrumententeils, also insbesondere der Instrumentenspitze, die Ausrichtung einer Ausspritzöffnung des Instruments, die Lage einer Schneidfläche, die Lage eines Entnahmefachs in einer Biopsienadel etc.

Falls das Instrument zum Injizieren eines Wirkstoffes gedacht ist, wird vorzugsweise aus der bestimmten Lage der Injektionsöffnung, die Wirkstoff-Injektionsrichtung berechnet. Dabei kann zur Beurteilung der Verteilung des Wirkstoffes die Lage des gesammten Instruments berücksichtigt werden, insbesondere zur Beurteilung des Rückflusses des Wirkstoffes entlang des Instrumentenpfades und des damit verbundenen Risikos des ungewünschten Austritts des Wirkstoffes aus dem Gewebe an eine Organoberfläche oder des Transports in ungewünschte Orte.

Um das erfindungsgemäße Verfahren durchzuführen, wird vorzugsweise zumindest auf einen Teil der Informationen zurückgegriffen, die im Folgenden erläutert werden. Hierbei handelt es sich insbesondere um hierin als "Körperstruktur-Daten", "Instrumenten-Daten" und "Bewegungs-Daten" bezeichnete Informationen, die vorzugsweise räumlich aufgelöst für die anisotrope Körperstruktur bereitgestellt werden. Insbesondere können sie sich von Ort zu Ort unterscheiden und insbesondere im Fall der Bewegungs-Daten zeitlich ändern.

Die Körperstruktur-Daten beschreiben insbesondere mechanische Eigenschaften der heterogenen Körperstruktur, die einen Einfluss auf die Bewegung des Instruments in der heterogenen Körperstruktur haben. Die Bewegung des Instruments in der heterogenen Körperstruktur wird durch eine mechanische Wechselwirkung zwischen dem Instrument und der heterogenen Körperstruktur beeinflusst. Die Körperstruktur-Daten erlauben es, die Auswirkung der Wechselwirkung auf die Bewegung zu bestimmen.

Beispiele für derartige Körperstruktur-Daten wurden bereits oben erwähnt. Die Körperstruktur-Daten werden vorzugsweise räumlich aufgelöst bereitgestellt. Insbesondere können sie sich von Ort zu Ort unterscheiden. Die Beispiele lauten wie folgt:
a) Ablenkeigenschaften. Sie beschreiben eine Ablenkung des Instruments bei Kontakt des Instruments mit der heterogenen Körperstruktur, also insbesondere lokal an einem Punkt oder Bereich der heterogenen Körperstruktur. Ablenkungen können hervorgerufen werden, wenn eine heterogenen Körperstruktur eine hohe Steifigkeit oder eine niedrige Penetrationsfähigkeit aufweist bzw. für das Instrument undurchdringlich ist. Die Ablenkeigenschaften können einem Abrutschen des Instruments an der harten Oberfläche oder zu einer Kippung der Bewegungsrichtung führen. Verwandt mit den Ablenkeigenschaften sind die im Folgenden erwähnten Biegeeigenschaften, die ebenfalls eine Bewegungsrichtungsänderung des Instruments bewirken können. Physikalische Größen zur Beschreibung der Ablenkeigenschaften sind insbesondere die (lokale) Härte der heterogenen Körperstruktur, beispielsweise beschreibbar durch VickersHärte- oder Martens-Härte etc., die Steifigkeit oder die Elastizität.
b) Formveränderungseigenschaften, insbesondere Biegeeigenschaften der heterogenen Körperstruktur sind ebenfalls durch die Härte und/oder Steifigkeit und/oder Formveränderbarkeit, insbesondere Elastizität der anisotropen Körperstruktur bedingt. Eine Formveränderbarkeit der heterogenen Körperstruktur bei Kontakt mit einem Instrument, das beispielsweise gebogen wird, kann zu einer Verlagerung der Bewegungsbahn des Instruments im Vergleich zu dem Fall führen, wenn die heterogenen Körperstruktur völlig steif ist. Je nach Formveränderbarkeit des Instruments, insbesondere Flexibilität und Biegbarkeit, ergeben sich dann bei Berücksichtigung der Bewegungs-Daten und der Instrumenten-Daten unterschiedliche Biegungen des Instruments entlang des Bewegungspfads. Diese können insbesondere basierend auf Gleichungen, die das Elastizitätsmodul des Instruments, geometrische Daten des Instruments, auf das Instrument wirkende Kräfte und Drehmomente sowie die vorgenannten Härte- und Elastizitätseigenschaften der anisotropen Körperstruktur berücksichtigen, berechnet werden. Der Krümmungsradius einer Biegung hängt dabei insbesondere vom Elastizitätsmoment und den auf das Instrument wirkenden Drehmomenten ab, sowie den geometrischen Kenndaten des Instruments, insbesondere des Durchmessers. Das Elastizitätsmodul (Young-Modul) ist wesentlich durch das Material des Instruments beeinflusst und kann abschnittsweise entlang des Instruments unterschiedlich sein. Andere Module, die die mechanischen Eigenschaften des Instruments oder der heterogenen Körperstruktur, insbesondere dessen oder deren Formveränderung unter Krafteinwirkung beschreiben können, sind z.B. das Kompressionsmodul, Dehnungsmodul, Schubmodul, Torsionsmodul, Spannungs-Dehnungsdiagramme etc.
c) Sperreigenschaften der heterogenen Körperstruktur. Wobei die Sperreigenschaften mechanische Eigenschaften beschreiben, die eine Vorwärtsbewegung des Instruments in der heterogenen Körperstruktur stoppen können. Die Sperreigenschaften werden insbesondere durch die Steifigkeit und/oder Durchdringbarkeit und/oder Härte eines Bereichs einer heterogenen Körperstruktur beschrieben. Dazu können ebenfalls Härtegrade und Härtedefinitionen, wie z.B. die Vickershärte oder Martenshärte verwendet werden. Auch kann natürlich auf Versuchsdaten zurückgegriffen werden, zu deren Erlangung die Durchdringbarkeit eines biologischen Gewebes mit vergleichbarer Sperreigenschaften (Penetrationsfähigkeit) mit Instrumenten getestet wurden. In Verbindung mit den Instrumentendaten und den Bewegungs-Daten kann dann bestimmt werden, ob die Penetrationsfähigkeit des Instruments und die z.B. während der Bewegung aufgewendeten Drücke und/oder Kräfte ausreichen, um die Sperreigenschaft der heterogenen Körperstruktur zu überwinden. Insbesondere kann es dazu kommen, dass das Instrument die Sperreigenschaften des Bereichs nicht überwinden kann und somit bei den gegebenen Bewegungs-Daten eine Weiterbewegung des Instruments blockiert wird. Alternativ kann es natürlich auch zu einer Knickung des Instruments führen, was ebenfalls basierend auf Instrumenten-Daten, insbesondere den Materialdaten und Kenndaten des Instruments und Geometriedaten des Instruments berechnet werden kann. Auch letzteres kann unerwünscht sein.

Beispiele für Instrumenten-Daten, die mechanische und/oder geometrische Eigenschaften des Instruments bestimmen, sind folgende:
a) Daten über die Formveränderbarkeit des Instruments, insbesondere die Elastizität und/oder Steifigkeit und/oder Härte und/oder Dehnbarkeit und/oder Bruchfestigkeit und/oder Knickfestigkeit des Instruments
b) Daten über die Geometrie und/oder Größe und/oder Form des Instruments. Insbesondere Daten über die Spitze des Instruments und die Form der Spitze, die insbesondre die Penetrationsfähigkeit des Instruments beeinflussen kann.
c) Daten über das Material des Instruments, die insbesondere einen wichtigen Einfluss auf die Elastizität des Instruments und Bruchfestigkeit des Instruments sowie Penetrationsfähigkeit des Instruments haben.

Beispiele für Bewegungs-Daten, die mechanische Eigenschaften der Bewegung beschreiben, sind:
a) Anfangsort der Bewegung;
b) Zielort der Bewegung;
c) die Kraft, die auf das Instrument wirkt, um eine Bewegung in der anisotropen Körperstruktur zu bewirken. Insbesondere die Richtung der Kraft und/oder der Betrag der Kraft. Insbesondere der Vektor der Kraft. Vorzugsweise, falls die Kraft zeitabhängig ist, die Kraft als Funktion der Zeit, insbesondere der Kraftvektor als Funktion der Zeit, insbesondere der Ort des Angriffspunktes der Kraft an das Instrument als Funktion der Zeit.
d) der Druck, der auf das Instrument wirkt, um eine Bewegung zu bewirken. Insbesondere die Richtung und/oder der Betrag des Drucks. Insbesondere falls der Druck zeitabhängig ist, die Zeitabhängigkeit des Drucks, insbesondere die Zeitabhängigkeit des Druckvektors, also der Druckvektor als Funktion der Zeit.
e) Momente, die auf das Instrument wirken, um es in der anisotropen Körperstruktur zu bewegen. Insbesondere Drehmomente und Kippmomente, die eine Bewegung des Instruments in der anisotropen Köperstruktur bewirken, insbesondere ein Vektor des Moments als Funktion der Zeit.
f) eine Bewegungsbahn, die sich ergeben würde, wenn die Bewegung durch eine homogene, insbesondere widerstandsfreie Körperstruktur verlaufen würde. Die Bewegungsbahn wird beispielsweise als Funktion der Lage des Instruments in Abhängigkeit von der Zeit, insbesondere der Lage der Instrumentenspitze in Abhängigkeit von der Zeit beschrieben. Hierbei kann auch angenommen werden, dass die Körperstruktur eine vorbestimmte homogene Viskosität aufweist, insbesondere widerstandslos ist. Eine Beeinflussung dieser als ungestört bezeichbaren Bewegungsbahn durch die heterogenen Eigenschaften der Körperstruktur kann dann basierend auf den Instrumenten-Daten und Körperstruktur-Daten berechnet werden.

Die vorgenannten Körperstruktur-Daten, Instrumenten-Daten und Bewegungs-Daten werden erfindungsgemäß vorzugsweise verwendet, um die Lage des Instruments in der heterogenen Körperstruktur zu bestimmen oder eine Wahrscheinlichkeit zu bestimmen, mit der diese Lage eingenommen wird. Dabei werden vorzugsweise mathematische Gleichungen eingesetzt, die die mechanische Wechselwirkung zwischen dem Instrument und der heterogenen Körperstruktur aufgrund der Bewegung des Instruments, die sich aus den Bewegungsdaten ergibt, beschreibt. Beispiele für derartige Gleichungen sind die physikalischen Newtongleichungen und Lagrangegleichungen zur Beschreibung einer Bewegung, bei dem insbesondere Zwangsbedingungen berücksichtigt werden können, die sich aus den Körperstruktur-Daten, Instrumenten-Daten und Bewegungs-Daten ergeben.

Mithilfe der vorgenannten Gleichungen und aufgrund numerischer Verfahren lässt sich insbesondere eine Beziehung zwischen der Lage des Instruments in der heterogenen Körperstruktur am Ende der Bewegung und der Lage des Instruments am Anfang der Bewegung bestimmen. Das Ende der Bewegung ist insbesondere dadurch gekennzeichnet, dass keine Kraft mehr auf das Instrument wirkt. Diese vorgenannte Beziehung wird also insbesondere basierend auf den Bewegungs-Daten, den Körperstruktur-Daten und den Instrumenten-Daten berechnet, und kann durch numerische Verfahren bestimmbar sein.

Vorzugsweise gibt ein Bediener, insbesondere ein Arzt oder Operateur eine gewünschte Lage des Instruments an. Basierend auf der vorgenannten bestimmten Beziehung lässt sich dann eine Soll-Anfangslage berechnen, die es erlaubt, die gewünschte Lage (wenn möglich) zu erreichen oder sie mit einer bestimmten Wahrscheinlichkeit zu erreichen. Dabei können mehrere Soll-Anfangslagen zu der gewünschten Lage führen. Insbesondere können basierend auf der vorgenannten Beziehung auch die Instrumenten-Daten variiert werden, um dadurch ein optimales Erreichen der gewünschten Lage zu erzielen. Beispielsweise kann die Steifigkeit des Instruments variiert werden, um schwer durchdringbare Bereiche der anisotropen Körperstruktur durchdringen zu können. Es kann aber auch die Elastizität des Instruments erhöht werden, um eine Knickgefahr des Instruments zu verringern und somit entlang eines gebogenen Pfades die gewünschte Lage zu erreichen. Beispielsweise schlängelt sich dann das Instrument geeigneter Elastizität an den rigiden Bereichen der anisotropen Körperstruktur entlang zu dem gewünschten Bereich. Insbesondere werden basierend auf der Beziehung auch die idealen Bewegungs-Daten berechnet, d.h. insbesondere der Kraftaufwand und die Kraftrichtung, die bei Vorwärtstreiben des Instruments aufgewendet werden sollen, um einerseits ein möglicherweise unerwünschtes Eindringen in rigide anisotrope Körperstrukturen zu vermeiden und andererseits ein Vorwärtstreiben des Instruments durch die heterogenen Körperstruktur durch ausreichenden Kraftaufwand zu ermöglichen.

Ein Bediener kann auch eine Steuerungsmechanik, insbesondere Automatik, vorzugsweise einen Roboterarm oder eine automatische oder semi-automatische Einbringungshilfe mit den erforderlichen Daten so programmieren, dass die zum Erreichen eines vorherbestimmten Zieles erforderlichen Bewegungs-, Instrumenten und Körperstruktur-Daten, oder eine Auswahl daraus genutzt werden, um ein Zielgebiet zu erreichen. Eine solche Automatik kann erfindungsgemäß die entsprechenden Daten auch direkt aus der Analysevorrichtung oder dem Planungsverfahren beziehen. Vorzugsweise wird zur Steuerung auf erfindungsgemäß berechnete Bewegungs-Daten zurückgegriffen, die sich für bestimmte Randbedingungen (Zwangsbedingungen) z.B. Endlage des Instruments und/oder gewünschten Bewegungspfad (zumindest gewünschte Abschnitte des Bewegungspfades) und/oder Vermeiden bestimmter Bereiche während der Bewegung und/oder mögliche Anfangslagen ergeben.

Vorzugsweise wird ein voraussichtlicher Bewegungspfad des Instruments in der heterogenen Körperstruktur von einer Anfangslage, die vorgegeben werden kann oder die als Soll-Anfangslage wie oben ausgeführt bestimmt wurde, zu der Endlage beschrieben. Die Endlage insbesondere die gewünschte Lage kann aber auch diejenige Lage sein, die sich aus einer vorgegebenen Anfangslage ergibt. Der Bewegungspfad wird vorzugsweise basierend auf den Bewegungs-Daten, den Körperstruktur-Daten und den Instrumenten-Daten berechnet, wobei mathematische Gleichungen, die die mechanischen Wechselwirkungen beschreiben und bereits oben erwähnt wurden, vorzugsweise verwendet werden. Dabei können insbesondere die Bewegungs-Daten und/oder Körperstruktur-Daten und/oder Instrumenten-Daten so optimiert werden, dass ein gewünschter oder akzeptabler Bewegungspfad, insbesondere zu einer gewünschten Endlage (ausgehend von einer möglichen Anfangslage) erzielt wird. '

Wie bereits oben ausgeführt wurde, werden erfindungsgemäß vorzugsweise auch Wahrscheinlichkeiten angegeben, dass z.B. eine bestimmte Lage erreicht wird oder ein bestimmtes Risiko besteht, dass eine Lage nicht erreicht wird. Vorzugsweise wird entlang des genannten voraussichtlichen Bewegungspfades zumindest abschnittsweise angegeben, wie hoch das Risiko ist, dass eine Abweichung von dem voraussichtlichen Bewegungspfad erfolgt. Alternativ oder zusätzlich kann ein wahrscheinlicher Bewegungskanal angegeben werden, innerhalb dem die Bewegung des Instruments mit einer vorbestimmten Wahrscheinlichkeit erfolgt. Die Wahrscheinlichkeiten können insbesondere basierend auf den Toleranzen berechnet werden, die für die Bewegungs-Daten und/oder Körperstruktur-Daten und/oder Instrumenten-Daten gelten und somit für eine Unsicherheit bei der Berechnung des Bewegungspfades sorgen. Beispiele sind Toleranzen beim Erzielen der Anfangsposition, Toleranzen beim Steuern der Kraft, die die Bewegung des Instruments in der anisotropen Körperstruktur bewirkt. Toleranzen der Materialkonstanten und der Geometrie des Instruments. Toleranzen der Steifigkeit und Elastizität in Bereichen der anisotropen Körperstruktur und der Penetrationsfähigkeit der anisotropen Körperstruktur usw. Der Begriff "Toleranzen" soll hierin insbesondere auch die Wahrscheinlichkeit der Abweichung von einem mittleren Wert umfassen.

Die vorliegende Erfindung betrifft weiter eine Vorrichtung, die eine Datenverarbeitungseinrichtung umfasst, die ausgebildet ist, basierend auf den Instrumenten-Daten, den Bewegungs-Daten und den Körperstruktur-Daten die Lage des Instruments in der anisotropen Körperstruktur oder die Wahrscheinlichkeit dieser Lage zu bestimmen, insbesondere durch Einsatz mathematischer Gleichungen und/oder numerischer Verfahren zu berechnen. Insbesondere weist die Vorrichtung eine Eingabeschnittstelle auf um die vorgenannten Daten einzugeben. Sie kann insbesondere auch eine Datenbank umfassen, in der die vorgenannten Daten zumindest teilweise gespeichert sind. Ebenfalls kann sie eine Diagnoseeinheit umfassen, die beispielsweise ausgebildet ist durch Elastographie, Körperstruktur-Daten zu erfassen. Insbesondere kann sie eine Überwachungseinheit, beispielsweise ein Röntgengerät umfassen, um die tatsächliche Lage des Instruments während des Einführvorgangs zu überwachen. Insbesondere kann sie ausgebildet Abweichungen der tatsächlichen Lage des Instruments von der gemäß dem erfindungsgemäßen Verfahren (als am wahrscheinlichsten) berechneten Lage des Instruments zu erkennen und entsprechende Warnsignale auszugeben und/oder daraus resultierend eine Änderung der Bewegungs-Daten zu erzeugen, die den aktuellen Einführvorgang steuern. Insbesondere weist sie eine mechanische Steuerung auf, die das Instrument automatisch steuert, um das Instrument in der heterogenen Körperstruktur voranzutreiben. Insbesondere umfasst sie das vorgenannte Instrument und insbesondere ein mechanische Einrichtung zum Bewegen des Instruments durch die heterogenen Körperstruktur.

Bei der folgenden detaillierten Beschreibung werden weitere Merkmale und Vorteile der Erfindung offenbart, die miteinander kombiniert werden können.

Figur 1 zeigt Einwirkungen der heterogenen Körperstruktur auf die Form des Instruments.

Figuren 2a bis 2g zeigen Beispiele, bei denen das Zielgebiet aufgrund der Wechselwirkung des Instrument mit der heterogenen Körperstruktur erreicht wird oder nicht erreicht wird.

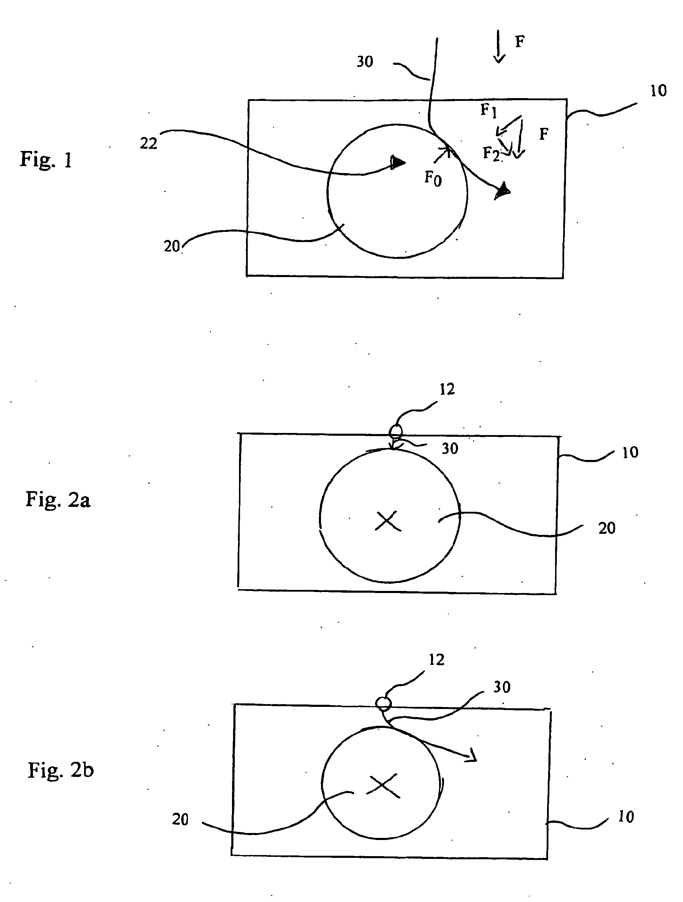

Figur 1 zeigt eine anisotrope Körperstruktur 10, ein Instrument 30, das in die heterogenen Körperstruktur 10 eingeführt wird. Ein Bereich 20 in der heterogenen Körperstruktur mit hoher Steifigkeit bzw. schwerer Durchdringbarkeit. Der Bereich innerhalb von 10 aber außerhalb von 20 soll leicht durchdringbar sein. Das Instrument 30 wird im Bereich 22 des starren Bereichs 20 von dem starren Bereich 20 abgelenkt und gebogen. Das Instrument 30 wird mit der Kraft F vorwärts geschoben. Die Kraft F kann nach dem Vektoradditionsgesetzen in eine Kraft F1 und eine Kraft F2 aufgeteilt werden. Die Kraft F1 erzeugt eine entsprechende Gegenkraft F0 im Bereich 22 der heterogenen Körperstruktur. Diese bewirkt eine Biegung des Instruments 30. Nach den Elastizitätsgesetzen ist die Krümmung der Biegung abhängig von dem Elastizitätsmodul des Instruments 30 sowie seinen geometrischen Eigenschaften, insbesondere dem Durchmesser. Das Elastizitätsmodul hängt insbesondere vom Material des Instruments ab. Basierend auf dem Krümmungsradius lässt sich der weitere Verlauf der Bewegung des Instruments der heterogenen Körperstruktur berechnen.

Im Bereich 22 des rigiden Bereichs 20 der heterogenen Körperstruktur kann es abhängig von den Formänderungseigenschaften des Bereichs 20 natürlich auch zu einer Verformung des Randes des rigiden Bereichs kommen, was wiederum berechnet werden kann und den Verlauf der Bewegungsbahn des Instruments 30 beeinflussen kann. Die Figuren 2a bis 2g zeigen Beispiele eines möglichen Bewegungsablaufes, der durch das erfindungsgemäße Verfahren berechnet, vorausgesagt und insbesondere geplant werden kann.

Mit einem Kreis 12 ist der Eintrittspunkt des Instruments in die heterogenen Körperstruktur jeweils in den Figuren bezeichnet. Das Kreuz x bezeichnet den gewünschten Zielbereich. Bei dem in Figur 2a gezeigten Beispiel ist der rigide Bereich 20 der heterogenen Körperstruktur zu starr oder zu wenig penetrationsfähig, um von dem Instrument 30 durchdrungen zu werden. Dieses Problem könnte beispielsweise durch Änderung der Instrumenten-Daten, also Erhöhung der Penetrationsfähigkeit des Instruments und/oder der Bewegungs-Daten (z.B. Änderung der Kraft) überwunden werden. Das Zielgebiet "x" kann somit bei dem Fall der Figur 2a, wie durch das erfindungsgemäße Verfahren berechnet bei den vorgegebenen Daten des Beispiels, nicht erreicht werden.

Bei dem in Figur 2b gezeigten Fall wird das Instrument 30 am rigiden Bereich 20 gebogen. Durch die Biegung des Instruments 30 ergibt sich eine Ablenkung der Spitze des Instruments weg von dem gewünschten Zielgebiet "x". Handelt es sich beispielsweise um einen Katheter, so würde im Falle der Figur 2b ein Ausspritzen des Wirkstoffes nicht den gewünschten Zielbereich "x" erreichen.

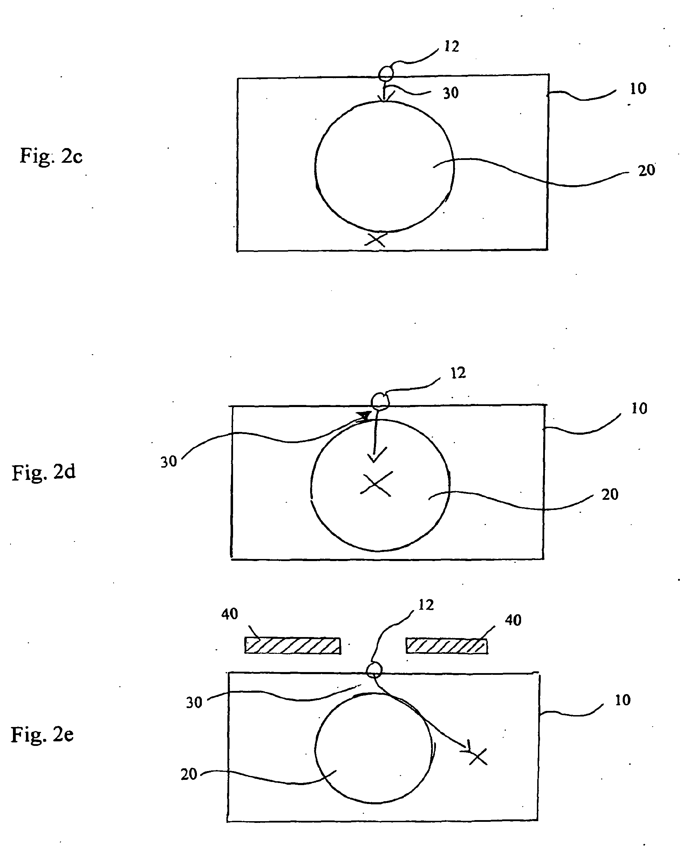

Bei dem in Figur 2c gezeigten Beispiel ist der rigide Bereich 20 ebenfalls nicht durchdringbar, so dass ein Operateur mit einem vorgesehenen Instrument (mit bestimmten Instrumenten-Daten und Bewegungs-Daten) das Zielgebiet x nicht erreichen kann. Es wird also blockiert.

Bei Figur 2d wurde mithilfe des erfindungsgemäßen Verfahrens ein Instrument mit geeigneten Instrumentendaten und geeignete Bewegungs-Daten ausgewählt, um den rigiden Bereich 20 zu durchdringen, um den gewünschten Zielort "x" zu erreichen.

Bei dem Beispiel der Figur 2e findet eine Biegung des Instruments 30 an dem rigiden Bereich 20 statt. Basierend auf den erfindungsgemäßen Verfahren wurden der geeignete Eintrittspunkt 20 und die geeigneten Instrumentendaten bestimmt, so dass sich das Instrument 30 in gewünschter Weise am rigiden Bereich 20 biegt, um so den Zielbereich "x" zu erreichen. Dies ist auch dann möglich, wenn der Anfangsort für die Bewegung 12 des Instruments 30 nur gering variabel ist, da Barrieren 40 ein Einführen des Instruments an einer anderen Stelle hindern. Die geeignete Wahl der Instrumenten-Daten, insbesondere die geeignete Elastizität des Instruments 12 (und auch der Bewegungs-Daten) erlaubt dann trotzdem den Zielbereich "x" zu erreichen. Insbesondere wird mithilfe des erfindungsgemäßen Verfahrens aus dem zum Erreichen des Zielpunktes "x" notwendigen Krümmungsradius des Instruments Elastizitätsdaten und Geometriedaten des Instruments berechnet, auf denen basierend ein geeignetes Instrument (automatisch) ausgewählt bzw. vorgeschlagen wird, das die passenden Kenndaten aufweist. Die Auswahl erfolgt beispielsweise aus einer Datenbank mit Instrumenten-Kenndaten.

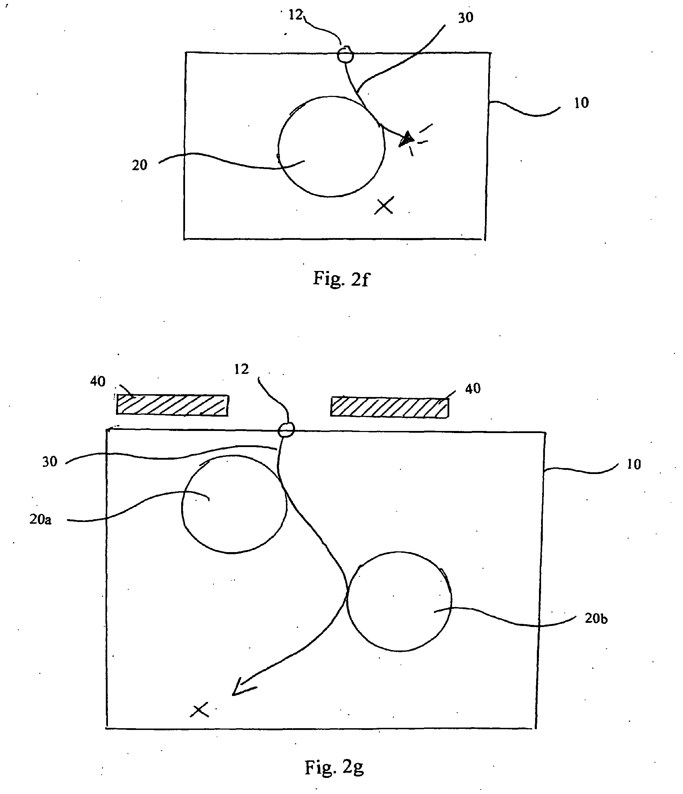

Die Figur 2f zeigt ein Beispiel, bei dem ein Instrument 30 durch die rigide Struktur 20 so abgelenkt wird, dass ein Ausspritzen des Wirkstoffes durch das Instrument 30 das Zielgebiet "x" verfehlt. Das Risiko hierfür kann mithilfe des erfindungsgemäßen Verfahrens berechnet werden.

Figur 2g zeigt ein Beispiel, wie mithilfe des erfindungsgemäßen Verfahrens ein gewünschter Zielbereich "x" erreicht werden kann. In der heterogenen Körperstruktur 10 befinden sich zwei rigide Bereiche 20a und 20b. Mögliche Anfangsorte für die Bewegung sind durch die Barrieren 40 verhindert. Eine geradlinige Bewegung des Instruments 30 in der Figur 2f würde dazu führen, dass das Instrument 30 gegen die rigide Oberfläche des Bereichs 20a stößt. Ein Durchdringen dieser Oberfläche ist aus medizinischen Gründen unerwünscht oder würde zu unerwünschten Instrumenteneigenschaften führen. Durch das erfindungsgemäße Verfahren kann eine geeignete Biegbarkeit des Instruments bestimmt werden, um den gewünschten Bewegungspfad zum Ziel "x" zu erreichen. Insbesondere kann ein Instrument mit passender Elastizität ausgewählt werden, das bei Kontakt mit den rigiden Bereichen 20a und 20b zu Krümmungsradien der Biegung des Instruments führt, so dass das Ende der Bewegungsbahn mit dem Zielbereich übereinstimmt.

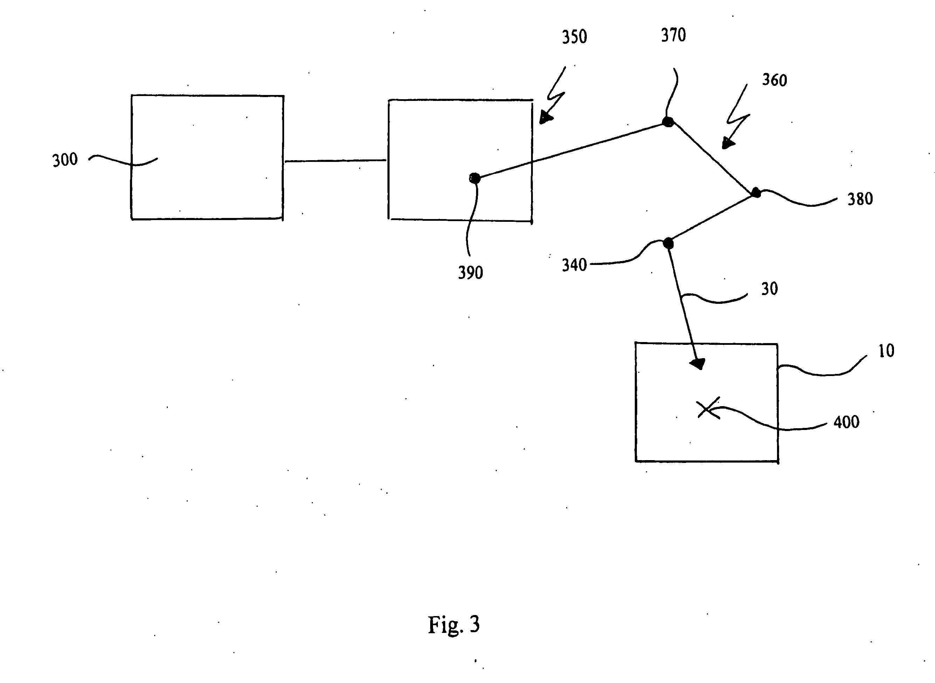

Figur 3 zeigt eine erfindungsgemäße Vorrichtung zum Steuern eines Instruments durch eine heterogene Körperstruktur, insbesondere eine Instrumentensteuervorrichtung. Eine Datenverarbeitungseinrichtung 300 berechnet Bewegungs-Daten zum Steuern einer Steuerungsmechanik basierend auf den Körperstruktur-Daten der heterogenen Körperstruktur 10, Instrumentendaten über ein Instrument 30 und einem gewünschten Zielgebiet 400 und/oder gewünschten Bewegungspfad.

Die Steuermechanik 350 umfasst einen Roboterarm 360 mit mehreren Gelenken 370, 380 und 390, der an seinem Ende 340 das Instrument 30 hält und es durch die heterogene Körperstruktur gemäß den berechneten Bewegungs-Daten für die Bewegung entlang einem Bewegungspfad zu dem gewünschten Zielgebiet 400 führt.

## Patentansprüche

1. Verfahren zur Planung der Lage und/oder eines Bewegungspfades eines medizinischen Instrumentes, z.B. eines Katheters, in einer heterogenen Körperstruktur, insbesondere zur Analyse der Risiken des Ablenkens oder Biegens eines Instrumentes weg von einem geplanten Einbringungspfad oder des Brechens des Instrumentes oder des Blockieren des Instrumentes auf dem geplanten Pfad und/oder zur Bestimmung der voraussichtlichen Lage des Instruments nach dem Einbringen des Instruments in die heterogene Körperstruktur
mit folgenden Schritten:
- Körperstruktur-Daten über mechanische Eigenschaften der heterogenen Körperstruktur, die eine Bewegung des Instruments durch die heterogene Körperstruktur auf Grund mechanischer Wechselwirkung zwischen dem Instrument (30) und der heterogenen Körperstruktur (10) beeinflussen, werden bereitgestellt;
- Instrumenten-Daten über mechanische und/oder geometrische Eigenschaften des Instruments werden bereitgestellt,
- Bewegungs-Daten über mechanische Eigenschaften, die eine Bewegung des Instruments (30) durch die heterogene Körperstruktur (10) bewirken sollen und/oder beschreiben, werden bereitgestellt,
- basierend auf den Bewegungs-Daten, den Körperstruktur-Daten und den Instrumenten-Daten wird die Lage und/oder der Bewegungspfad des Instruments (30) in der anisotropen Körperstruktur oder die Wahrscheinlichkeit dieser Lage und/oder dieses Bewegungspfades bestimmt.

2. Verfahren nach Anspruch 1, bei welchem eine Beziehung zwischen der Lage des Instruments (30) in der heterogenen Körperstruktur (10) am Ende der Bewegung und der Lage des Instruments am Anfang der Bewegung basierend auf den Bewegungs-Daten, den Körperstruktur-Daten und den Instrumenten-Daten berechnet wird.

3. Verfahren nach Anspruch 2, bei welchem basierend auf der Beziehung und einer gewünschten Lage des Instruments (30) Soll-Bewegungs-Daten, insbesondere eine Soll-Anfangslage und/oder Soll-Instrumenten-Daten bestimmt werden, die zum Erreichen der gewünschten Lage und/oder zum Verfolgen des gewünschten Bewegungspfades notwendig sind und/oder die Wahrscheinlichkeit des Erreichens erhöhen.

4. Verfahren nach einem der vorhergehenden Ansprüche, insbesondere nach Anspruch 3, bei welchem ein voraussichtlicher Bewegungspfad des Instruments (30) in der Körperstruktur, von einer Anfangslage, insbesondere Soll-Anfangslage zu der Endlage, insbesondere gewünschten Lage basierend auf den Bewegungs-Daten, den Körperstruktur-Daten und den Instrumenten-Daten berechnet wird.

5. Verfahren nach Anspruch 4, bei welchem zumindest für Abschnitte des Bewegungspfades das Risiko der Abweichung vom voraussichtlichen Bewegungspfad, insbesondere basierend auf gegebenen Toleranzen der Bewegungs-Daten und/oder Körperstruktur-Daten und/oder Instrumenten-Daten berechnet wird und/oder basierend auf den Toleranzen ein Bewegungskanal berechnet wird, in dem mit einer vorbestimmten Wahrscheinlichkeit die Bewegung des Instruments (30) erfolgt.

6. Verfahren nach einem der vorhergehenden Ansprüche, bei welchem die Köperstruktur-Daten zumindest eine der folgenden mechanischen Eigenschaften der heterogenen Körperstruktur bestimmen:
- Ablenkeigenschaften, die eine Ablenkung des Instruments bei Kontakt des Instruments mit einem Bereich der anisotropen Körperstruktur beschreiben;
- Formveränderungseigenschaften, die eine Formveränderung des Instruments bei Kontakt des Instruments mit einem Bereich der anisotropen Körperstruktur beschreiben;
- Sperreigenschaften der anisotropen Körperstruktur, wobei die Sperreigenschaften eine Vorwärtsbewegung des Instruments in der anisotropen Körperstruktur stoppen können, wobei die Fähigkeit zum Stoppen der Bewegung von der Penetrationsfähigkeit des Instruments abhängt;
- Viskosität der heterogenen Körperstruktur.

7. Verfahren nach einem der vorhergehenden Ansprüche, bei welchem die Instrumenten-Daten zumindest eine der folgenden mechanischen Eigenschaften des Instruments bestimmen:
- Formveränderbarkeit des Instruments, insbesondere Flexibilität und/oder Elastizität und/oder Steifigkeit und/oder Härte und/oder Dehnbarkeit und/oder Bruchfestigkeit und/oder Knickfestigkeit des Instruments;
- Geometrie und/oder Größe und/oder Form des Instruments;
- Daten über das Material oder die Materialien des Instruments.

8. Verfahren nach einem der vorhergehenden Ansprüche, bei welchem die Bewegungs-Daten über mechanische Eigenschaften, die eine Bewegung des Instruments durch die heterogene Körperstruktur bewirken sollen und/oder beschreiben, zumindest eine der folgenden mechanischen Eigenschaften der Bewegung bestimmen:
- Anfangsort der Bewegung;
- Zielort der Bewegung;
- Kraft, die die Bewegung bewirken soll, insbesondere Richtung und/oder Betrag der Kraft, insbesondere Kraftvektor als Funktion der Zeit;
- Druck, der die Bewegung bewirken soll, insbesondere Richtung und/oder Betrag des Drucks, insbesondere Druckvektor als Funktion der Zeit;
- Momente, insbesondere Drehmomente, die eine Bewegung des Instruments, insbesondere Kippung und/oder Drehung bewirken sollen, insbesondere Momentenvektor als Funktion der Zeit;
- Impulse, die auf das Instrument wirken, insbesondere Impulsvektor als Funktion der Zeit;
- ein Bewegungspfad, der den Verlauf der vorgesehenen Bewegung beschreiben würde,
wenn die Bewegung durch eine homogene Körperstruktur insbesondere vorbestimmter Viskosität verlaufen würde.

9. Computerprogramm, das, wenn es in einem Computer geladen wird oder wenn es auf einem Computer läuft, den Computer veranlasst, das Verfahren nach einem der Ansprüche 1 bis 8 durchzuführen.

10. Vorrichtung zum Bestimmen der Lage und/oder eines Bewegungspfades eines Instruments in einer heterogenen Körperstruktur und/oder zum Einführen eines Instruments in eine heterogene Körperstruktur und/oder zum Planen eines Bewegungspfades, insbesondere des Einführpfades eines Instruments in eine heterogene Körperstruktur, wobei die Vorrichtung eine Datenverarbeitungseinrichtung umfasst, die ausgebildet ist, basierend auf Instrumenten-Daten, Bewegungs-Daten und Körperstruktur-Daten die Lage und/oder den Bewegungspfad des Instruments unter Verwendung der Schritte nach einem der Verfahren nach Anspruch 1 bis 8 zu bestimmen und/oder zu planen und/oder zu steuern.

11. Vorrichtung nach Anspruch 10, wobei die Vorrichtung eine Steuereinrichtung zur mechanischen Steuerung des Instruments umfasst, um ein Instrument gemäß dem bestimmten und/oder geplanten Bewegungspfad durch die heterogene Körperstruktur insbesondere automatisch zu bewegen.

12. Vorrichtung nach Anspruch 10 oder 11, bei welchem die Vorrichtung eine Auswahleinrichtung umfasst, die insbesondere automatisch aus einem gegebenen Satz von Instrumenten ein geeignetes Instrument auswählt, um einen mithilfe der Datenverarbeitungseinrichtung geplanten oder bestimmten Bewegungspfad durch die heterogene Körperstruktur mit der Steuereinrichtung, die das Instrument steuert, steuern zu können.

13. Vorrichtung nach Anspruch 11 oder 12, bei welchem die Steuereinrichtung einen Roboter umfasst, der die Bewegung des Instruments entsprechend den Bewegungs-Daten steuert, die für den bestimmten und/oder geplanten Bewegungspfad bestimmt wurden.

14. Instrumentensteuervorrichtung zum Steuern eines Instruments durch eine heterogene Körperstruktur mit einer Steuerungsmechanik, insbesondere einem Roboter, der die Bewegung des Instruments im Raum steuert;
mit einer Datenverarbeitungseinrichtung, die basierend auf Körperstruktur-Daten über mechanische Eigenschaften der heterogenen Körperstruktur, Instrumenten-Daten über mechanische und/oder geometrische Eigenschaften des Instruments und einer gewünschten Endlage des Instruments in der heterogenen Körperstruktur Bewegungs-Daten über mechanische Eigenschaften der Bewegung des Instruments in der heterogenen Körperstruktur berechnet, um die Endlage zu erreichen, wobei die Steuermechanik ausgebildet ist, gemäß den berechneten Bewegungs-Daten gesteuert zu werden.

## Claims

1. A method for planning the position and/or a movement trajectory of a medical instrument, e.g. a catheter, in a heterogeneous body structure, in particular for analysing the risks of an instrument deviating or bending away from a planned insertion trajectory or of the instrument breaking or being blocked on the planned trajectory and/or for determining the expected position of the instrument after the Instrument has been inserted into the heterogeneous body structure, comprising the steps of:
- providing body structure data concerning mechanical properties of the heterogeneous body structure which influence a movement of the instrument through the heterogeneous body structure due to the mechanical interaction between the instrument (30) and the heterogeneous body structure (10);
- providing instrument data concerning mechanical and/or geometric properties of the instrument;
- providing movement data concerning mechanical properties which are intended to cause and/or which describe a movement of the instrument (30) through the heterogeneous body structure (10);
- determining the position and/or movement trajectory of the instrument (30) in the anisotropic body structure or the probability of said position and/or movement trajectory, on the basis of the movement data, the body structure data and the instrument data.

2. The method according to claim 1, wherein a relationship between the position of the instrument (30) in the heterogeneous body structure (10) at the end of the movement and the position of the instrument at the start of the movement is calculated on the basis of the movement data, the body structure data and the instrument data.

3. The method according to claim 2, wherein on the basis of the relationship and a desired position of the instrument (30), nominal movement data - in particular a nominal starting position - and/or nominal instrument data are determined which are necessary for reaching the desired position and/or for tracking the desired movement trajectory and/or which increase the probability of reaching it.

4. The method according to any one of the preceding claims, in particular according to claim 3, wherein an expected movement trajectory of the instrument (30) in the body structure from a starting position, in particular a nominal starting position, to the end position, in particular the desired position, is calculated on the basis of the movement data, the body structure data and the instrument data.

5. The method according to claim 4, wherein the risk of deviating from the expected movement trajectory is calculated, at least for sections of the movement trajectory, and in particular on the basis of given tolerances of the movement data and/or body structure data and/or instrument data, and/or a movement channel in which the instrument (30) is moved with a predetermined probability is calculated on the basis of the tolerances.

6. The method according to any one of the preceding claims, wherein the body structure data determine at least one of the following mechanical properties of the heterogeneous body structure:
- deviating properties which describe a deviation of the instrument when the instrument contacts a region of the anisotropic body structure;
- shape-changing properties which describe a change in the shape of the instrument when the instrument contacts a region of the anisotropic body structure;
- blocking properties of the anisotropic body structure, wherein the blocking properties can stop a forward movement of the instrument in the anisotropic body structure, and wherein their ability to stop the movement depends on the penetration of the instrument;
- the viscosity of the heterogeneous body structure.

7. The method according to any one of the preceding claims, wherein the instrument data determine at least one of the following mechanical properties of the instrument:
- the malleability of the instrument, in particular the flexibility and/or elasticity and/or rigidity and/or hardness and/or distensibility and/or tensile strength and/or buckling resistance of the instrument;
- the geometry and/or size and/or shape of the instrument;
- data concerning the material or materials of the instrument.

8. The method according to any one of the preceding claims, wherein the movement data concerning mechanical properties, which are intended to cause and/or which describe a movement of the instrument through the heterogeneous body structure, determine at least one of the following mechanical properties of the movement:
- the starting location of the movement;
- the target location of the movement;
- the force which is intended to cause the movement, in particular the direction and/or the magnitude of the force, in particular the force vector as a function of time;
- the pressure which is intended to cause the movement, in particular the direction and/or magnitude of the pressure, in particular the pressure vector as a function of time;
- moments, in particular torques, which are intended to move the instrument, in particular tilt and/or rotate it, in particular a moment vector as a function of time;
- impulses which act on the instrument, in particular an impulse vector as a function of time;
- a movement trajectory which would describe the course of the planned movement if the movement passed through a homogeneous body structure having in particular a predetermined viscosity.

9. A computer program which, when it is loaded onto a computer or is running on a computer, causes the computer to perform the method according to any one of claims 1 to 8.

10. A device for determining the position and/or a movement trajectory of an instrument in a heterogeneous body structure and/or for inserting an instrument into a heterogeneous body structure and/or for planning a movement trajectory, in particular the insertion trajectory of an instrument into a heterogeneous body structure, wherein the device includes a data processing means designed to determine and/or plan and/or guide the position and/or movement trajectory of the instrument on the basis of instrument data, movement data and body structure data, using the steps according to any one of the methods according to claims 1 to 8.

11. The device according to claim 10, wherein the device includes a guiding means for mechanically guiding the instrument in order to move an instrument, in particular automatically, through the heterogeneous body structure in accordance with the determined and/or planned movement trajectory.

12. The device according to claim 10 or 11, wherein the device includes a selecting means which selects, in particular automatically, a suitable instrument from a given set of instruments, in order to be able to guide a movement trajectory, planned or determined with the aid of the data processing means, through the heterogeneous body structure using the guiding means which guides the instrument.

13. The device according to claim 11 or 12, wherein the guiding means includes a robot which guides the movement of the instrument in accordance with the movement data determined for the determined and/or planned movement trajectory.

14. An instrument guiding device for guiding an instrument through a heterogeneous body structure, comprising: a guiding mechanism, in particular a robot, which guides the spatial movement of the instrument; and a data processing means which calculates movement data concerning mechanical properties of the movement of the instrument in the heterogeneous body structure on the basis of body structure data concerning mechanical properties of the heterogeneous body structure, instrument data concerning mechanical and/or geometric properties of the instrument and a desired end position of the instrument in the heterogeneous body structure, in order to reach the end position, wherein the guiding mechanism is designed to be guided in accordance with the calculated movement data.

## Revendications

1. Procédé pour planifier la position et/ou une trajectoire de mouvement d'un instrument médical, par exemple d'un cathéter, dans une structure corporelle hétérogène, en particulier pour analyser les risques de déviation ou de flexion d'un instrument s'éloignant d'une trajectoire d'introduction planifiée ou de rupture de l'instrument ou de blocage de l'instrument sur la trajectoire planifiée et/ou pour déterminer la position prévisible de l'instrument après l'introduction de l'instrument dans la structure corporelle hétérogène
comportant les étapes suivantes consistant à :
- fournir des données de structure corporelle concernant des propriétés mécaniques de la structure corporelle hétérogène, qui influent sur un mouvement de l'instrument à travers la structure corporelle hétérogène en raison de l'interaction mécanique entre l'instrument (30) et la structure corporelle hétérogène (10),
- fournir des données d'instrument concernant des propriétés mécaniques et/ou géométriques de l'instrument,
- fournir des données de mouvement concernant des propriétés mécaniques qui doivent provoquer et/ou qui décrivent un mouvement de l'instrument (30) à travers la structure corporelle hétérogène (10),
- sur la base des données de mouvement, des données de structure corporelle et des données d'instrument, déterminer la position et/ou la trajectoire de mouvement de l'instrument (30) dans la structure corporelle anisotrope ou la probabilité d'atteindre cette position et/ou d'obtenir cette trajectoire de mouvement.

2. Procédé selon la revendication 1, dans lequel une relation entre la position de l'instrument (30) dans la structure corporelle hétérogène (10) à la fin du mouvement et la position de l'instrument au début du mouvement est calculée sur la base des données de mouvement, des données de structure corporelle et des données d'instrument.

3. Procédé selon la revendication 2, dans lequel des données de consigne de mouvement, en particulier une position initiale de consigne et/ou des données de consigne d'instrument sont déterminées sur la base de la relation et d'une position désirée de l'instrument (30), lesquelles données sont nécessaires pour atteindre la position désirée et/ou pour suivre la trajectoire de mouvement désirée, et/ou qui accroissent la probabilité d'atteindre cette position ou de suivre cette trajectoire.

4. Procédé selon l'une quelconque des revendications précédentes, en particulier selon la revendication 3, dans lequel une trajectoire de mouvement prévue de l'instrument (30) dans la structure corporelle est calculée à partir d'une position initiale, en particulier de la position initiale de consigne jusqu'à la position finale, en particulier la position désirée, sur la base des données de mouvement, des données de structure corporelle et des données d'instrument.

5. Procédé selon la revendication 4, dans lequel au moins pour des portions de la trajectoire de mouvement, le risque d'écartement de la trajectoire de mouvement prévue est calculé, en particulier sur la base de tolérances fournies pour les données de mouvement et/ou les données de structure corporelle et/ou les données d'instrument, et/ou est calculé sur la base des tolérances d'un canal de mouvement, dans lequel le mouvement de l'instrument (30) s'effectue avec une probabilité déterminée.

6. Procédé selon l'une quelconque des revendications précédentes, dans lequel les données de structure corporelle déterminent au moins une des propriétés mécaniques suivantes de la structure corporelle hétérogène :
- des propriétés de déviation qui décrivent une déviation de l'instrument lorsque l'instrument entre en contact avec une région de la structure corporelle anisotrope,
- des propriétés de déformation qui décrivent une déformation de l'instrument lorsque l'instrument entre en contact avec une région de la structure corporelle anisotrope,
- des propriétés de blocage de la structure corporelle anisotrope, dans lequel les propriétés de blocage peuvent arrêter un mouvement d'avance de l'instrument dans la structure corporelle anisotrope, dans lequel la capacité d'arrêt du mouvement dépend de la capacité de pénétration de l'instrument,
- la viscosité de la structure corporelle hétérogène.

7. Procédé selon l'une quelconque des revendications précédentes, dans lequel les données d'instrument déterminent au moins une des propriétés mécaniques suivantes de l'instrument :
- la capacité de déformation de l'instrument, en particulier la souplesse et/ou l'élasticité et/ou la rigidité et/ou la dureté et/ou l'extensibilité et/ou la résistance à la traction et/ou la résistance au flambage de l'instrument,
- la géométrie et/ou la taille et/ou la forme de l'instrument,
- des données concernant le matériau ou les matériaux de l'instrument.

8. Procédé selon l'une quelconque des revendications précédentes, dans lequel les données de mouvement concernant des propriétés mécaniques qui doivent provoquer et/ou qui décrivent un mouvement de l'instrument à travers la structure corporelle hétérogène, déterminent au moins une des propriétés mécaniques suivantes du mouvement :
- l'emplacement de départ du mouvement,
- l'emplacement d'arrivée du mouvement,
- la force destinée à produire le mouvement, en particulier la direction et/ou la grandeur de la force, en particulier le vecteur de force en fonction du temps,
- la pression destinée à produire le mouvement, en particulier la direction et/ou la grandeur de la pression, en particulier le vecteur de pression en fonction du temps,
- les moments, en particulier les couples de rotation, destinés à produire un mouvement de l'instrument, en particulier un basculement et/ou une rotation, en particulier le vecteur de moment en fonction du temps,
- impulsions agissant sur l'instrument, en particulier vecteur d'impulsion en fonction du temps,
- une trajectoire de mouvement qui décrit la courbe du mouvement prévu lorsque le mouvement s'effectue à travers une structure corporelle homogène, en particulier ayant une viscosité prédéterminée.

9. Programme informatique qui, lorsqu'il est chargé dans un ordinateur ou lorsqu'il s'exécute sur un ordinateur, amène l'ordinateur à mettre en oeuvre le procédé selon l'une quelconque des revendications 1 à 8.

10. Dispositif pour déterminer la position et/ou la trajectoire de mouvement d'un instrument dans une structure corporelle hétérogène et/ou pour insérer un instrument dans une structure corporelle hétérogène et/ou pour planifier une trajectoire de mouvement, en particulier la trajectoire d'introduction d'un instrument dans une structure corporelle hétérogène, dans lequel le dispositif inclut un dispositif de traitement de données qui est configuré pour, sur la base de données d'instrument, de données de mouvement et de données de structure corporelle, déterminer et/ou planifier et/ou commander la position et/ou la trajectoire de mouvement de l'instrument en utilisant les étapes selon l'un quelconque des procédés selon les revendications 1 à 8.

11. Dispositif selon la revendication 10, dans lequel le dispositif inclut un dispositif de commande pour commander mécaniquement, en particulier automatiquement, l'instrument afin de déplacer un instrument selon la trajectoire de mouvement déterminée et/ou planifiée à travers la structure corporelle hétérogène.

12. Dispositif selon la revendication 10 ou 11, dans lequel le dispositif inclut un dispositif de sélection qui sélectionne, en particulier automatiquement, un instrument adapté parmi un ensemble donné d'instruments, pour pouvoir commander une trajectoire de mouvement planifiée ou déterminée à travers la structure corporelle hétérogène avec les moyens de commande qui commandent l'instrument, à l'aide des moyens de traitement de données.

13. Dispositif selon la revendication 11 ou 12, dans lequel les moyens de commande incluent un robot qui commande le mouvement de l'instrument en fonction des données de mouvement, lesquelles données ont été déterminées pour la trajectoire de mouvement déterminée et/ou planifiée.

14. Dispositif de commande d'instrument pour commander un instrument à travers une structure corporelle hétérogène avec un mécanisme de commande, en particulier un robot, qui commande le mouvement de l'instrument dans l'espace,
comportant des moyens de traitement de données qui, sur la base de données de structure corporelle concernant des propriétés mécaniques de la structure corporelle hétérogène, de données d'instrument concernant des propriétés mécaniques et/ou géométriques de l'instrument et d'une position finale désirée de l'instrument dans la structure corporelle hétérogène, calculent des données de mouvement concernant des propriétés mécaniques du mouvement de l'instrument dans la structure corporelle hétérogène, afin d'atteindre la position finale, dans lequel le mécanisme de commande est configuré pour être commandé en fonction des données de mouvement calculées.
